# Europäisches Patentamt

## European Patent Office

## Office européen des brevets

(11) Publication number: **0 205 532**
**B1**

# EUROPEAN PATENT SPECIFICATION

(45) Date of publication of the patent specification:
27.12.89

(21) Application number: **86900213.9**

(22) Date of filing: **19.12.85**

(86) International application number:
**PCT/GB 85/00591**

(87) International publication number:
**WO 86/03782 (03.07.86 Gazette 86/14)**

(51) Int. Cl.⁴: **C 12 Q 1/68,** G 01 N 33/546 //
G01N33/548

(54) **IMPROVED SANDWICH HYBRIDISATION TECHNIQUE FOR THE DETECTION OF NUCLEOTIDE SEQUENCES.**

(30) Priority: **19.12.84 GB 8432118**

(43) Date of publication of application:
**30.12.86 Bulletin 86/52**

(45) Publication of the grant of the patent:
**27.12.89 Bulletin 89/52**

(84) Designated Contracting States:
**BE CH DE FR IT LI NL SE**

(56) References cited:
**EP-A-0 070 687**
**EP-A-0 079 139**
**EP-A-0 124 221**
**EP-A-0 130 515**
**EP-A-0 130 523**
**WO-A-84/04332**
**US-A-4 395 486**

**CHEMICAL ABSTRACTS, vol. 96, no. 23, 07 June 1982, Columbus, OH (US); B.SEED: "Diazotizable arylamine cellulose papers for the coupling and hybridization of nucleic acids", p. 356, no. 196141w
PROCEEDINGS of the National Academy of Science, USA, vol. 80, Jan 1983; B.J.CONNER et al.: "Detection of sickle cell beta-s-globin allele by hybridization with synthetic oligonucleotides", pp. 278-282**

**The file contains technical information submitted**

(73) Proprietor: **Malcolm, Alan David Blair, 22 Elm Crescent, Ealing London, W5 3JW (GB)**
Proprietor: **LANGDALE, Jane Alison, 10A Somerset Road, Ealing, London W13 (GB)**

(72) Inventor: **Malcolm, Alan David Blair, 22 Elm Crescent, Ealing London, W5 3JW (GB)**
Inventor: **LANGDALE, Jane Alison, 10A Somerset Road, Ealing, London W13 (GB)**

(74) Representative: **Lambert, Hugh Richmond, D. YOUNG & CO. 10 Staple Inn, London, WC1V 7RD (GB)**

(56) References cited: (continuation)
**after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).

EP 0 205 532 B1

## Description

### Field of Invention

This invention relates to the detection of nucleotide sequences, including both DNA and RNA sequences.

### Background and Prior Art

The ability to detect the presence (or absence) of a given RNA or DNA sequence in a sample, e.g. a clinically obtained sample or specimen, is potentially of great benefit to mankind. Existing methods of detection are, however, extremely time consuming and labour intensive and are therefore unsuitable for use on anything other than a very limited scale. In many cases also sensitivity is undesirably low. In one commonly used procedure, for example, specific DNA sequences are detected in complex mixtures by digesting the sample with an appropriate restriction endonuclease, followed by size fractionation by gel electrophoresis, transfer of the sized fractions from the gel to a nitrocellulose or other suitable membrane, hybridisation to a radioactively labelled probe, usually a $^{32}$P-labelled probe, and finally autoradiography. Such a procedure is inherently cumbersome and unsuited for automation. Also, very often the quantity of DNA or other nucleotide sequence to be detected and which is transferred onto the membrane is so small that in order to obtain a detectable signal after hybridisation a highly radioactive probe has to be used, which is disadvantageous both from the handling point of view and from the point of view that the radioactive label of choice, i.e. $^{32}$P, has a relatively short half-life making it unsuitable for use as a stock reagent.

An alternative to the above is the sandwich hybridisation technique disclosed by Dunn A.R. and Hassell J.A. in (1977) Cell *12* 23 - 26 in which the sample containing the nucleotide sequence to be detected is contacted with a complementary fragment immobilised on a membrane, e.g. a nitrocellulose filter, and to which the sequence to be detected hybridises in a first hybridisation step leaving an unhybridised tail available for a second hybridisation step with a labelled probe.

In EP-0 079 139 a proposal is made for the utilisation of the above described sandwich hybridisation technique in the identification of microorganisms in a sample. In accordance with that proposal a sample containing a single-stranded nucleic acid sequence from the micro-organism to be detected is contacted with two single-stranded fragments obtained from the genome of the micro-organism in question either directly or by recombinant DNA technology and complementary to different positions of the sequence to be detected, but not to each other, the one being immobilised on a solid carrier, preferably a nitrocellulose filter, and the other being labelled. Although labelling with labels other than radioactive labels is implied, only radioactive labelling is disclosed, and specifically labelling with $^{125}$I. In the presence of the single-stranded nucleic acid sequence to be detected, which hybridises with both the immobilised fragment and the labelled fragment, the labelled fragment becomes bound to the carrier and can be detected thereon by autoradiography, thus giving rise to positive identification of the microorganism from which that single-stranded sequence originated.

Whilst the sandwich hybridisation technique provides advantages over the first described procedure inherent disadvantages remain arising particularly from the finite and quite limited quantity of nucleic acid which can be immobilised onto the nitrocellulose filter or other membrane. This in particular places limits on both the speed and sensitivity of the detection method. Also it appears that the efficiency of the hybridisation particularly in the second stage is very low, thus reducing the sensitivity of the method still further.

In EP-0 070 687 a hybridisation diagnostic method is disclosed which uses light-labelled single-stranded polynucleotide reagents for hybridising with immobilised sample single-stranded polynucleotides. A variety of solid supports are suggested for the immobilised sample single-stranded polynucleotide including activated glass beads, polyacrylamide, agarose or Sephadex® beads, and cellulose. Various known methods are also suggested for coupling the sample polynucleotide to the support, largely by reference to published literature, e.g. Methods in Enzymology Vol. XXXIV, part B, 463 - 475, 1974 and Vol. XLIV, 859 - 886, 1976, but no specific examples of an operative method are given. It is also suggested that the sample singlestranded polynucleotide can be immobilised by contacting the sample under hybridisation conditions with an immobilised first single-stranded polynucleotide reagent complementary to a different and mutually exclusive portion of the sample polynucleotide to that which is required for the hybridisation of the light-labelled polynucleotide reagent, i.e. a so-called sandwich hybridisation procedure. Again no specific examples are given of the procedure, or of the method by which the first polynucleotide reagent is attached to the support in the first place.

### Summary of the Invention

In accordance with one aspect of the present invention we have discovered that both the speed and efficiency of the sandwich hybridisation reaction, particularly the second stage, can be substantially increased by using solid particles or beads of N,N'-methylene bisacrylamide cross-linked allyl dextran (Registered Trade Mark SEPHACRYL) as the carrier for the immobilised nucleic acid fragment thus providing a method for the detection of nucleic acid fragments which is capable of providing a much improved sensitivity. In particular the use of particles or beads of Sephacryl® appears to result in substantially improved covalent bonding of the polynucleotide reagent to the support.

In a second aspect of this invention, a particularly sensitive method has been developed for the detection of the abnormal human (sickle cell) β-globin gene by the sandwich hybridisation of a Dde I digest of the abnormal

2

gene with an immobilised polynucleotide reagent covalently bonded to Sephacryl® beads, and a second labelled polynucleotide reagent, such reagents comprising polynucleotide sequences obtained by restriction endonuclease treatment of the normal gene and complementary to different portions of a restriction fragment contained within the digest and containing the single base change in the sixth codon of the β-globin gene that is characteristic of the abnormal (sickle cell) β-globin gene.

In accordance with the present invention therefore, there is provided a method for the detection of a given nucleic acid sequence in a sample, which comprises contacting the sample under hybridisation conditions with a first reagent comprising solid beads of Sephacryl® having immobilized thereon a single-stranded nucleic acid fragment comprising a sequence complementary to a portion of the sequence to be detected and with a second reagent comprising a labelled singlestranded nucleic acid fragment comprising a sequence complementary to a different portion of the sequence to be detected, but non-complementary to the immobilised fragment, thereby forming a hybridisation duplex comprising the sequence to be detected hybridised with both reagents, separating the hybridisation duplex from any remaining unbound label, and detecting the presence, if any, of label bound to the hybridisation duplex.

## Detailed Description

The first reagent used in the method of this invention comprises a single-stranded nucleic acid fragment immobilised onto the Sephacryl® beads by covalent bonding between reactive, e.g. amino, groups in the nucleic acid fragment, and reactive groups in or on the solid particle.

Various methods exist and are known for the covalent bonding of nucleic acid fragments to such polymer beads, including coupling with carbodiimides via terminal phosphate groups and coupling via cyanogen bromide. Particular mention may be made, however, of coupling via diazotisation of aromatic amino groups attached to the polymer matrix, which technique is disclosed in detail in (1982) Nucleic Acid Res. 10, 22, 1799 - 1810 and 7163 - 7196.

Preferably the polymer beads will have a particle size in the range 5 to 50 μ, more preferably 10 to 20 μ.

Likewise various methods exist and are known for the labelling of single-stranded nucleic acid fragments to provide the second reagent used in the method of this invention. Labelling with radioactive isotopes such as $^{32}P$ and $^{125}P$ has already been mentioned and can be used in the present invention. However, it is envisaged that, because of the greater sensitivity that can be achieved by the present invention, less highly radioactive isotopes can be used as the label and in particular tritium. Also it is envisaged that non-radioactive labels e.g. enzymatic, fluorescent and chemiluminescent labels will be suitable, and in many cases may be preferred.

Once the reagents have been prepared, and it is envisaged that these will be available in kit form, the method of the invention is extremely simple to carry out and is readily adaptable to automatic or semi-automatic equipment. Essentially it is necessary merely to mix the sample with the two reagents, either simultaneously or successively, under conditions effective to permit formation of the hybridised duplex comprising the two reagents hybridised to the restriction fragment which is to be detected. The hybridisation is rapid, and since in contrast to the sandwich hybridisation technique of EP-0 079 139, both the first (immobilised) reagent and the second (labelled) reagent can be used to infinite excess, merely by increasing the quantity used, the amount of bound label can be maximised thereby to provide a maximum signal for subsequent detection. Thus the method of the present invention can be of great sensitivity, this being determined not by the concentration of the reagents, which effectively can be infinite, but by the concentration of the sequence to be detected in the sample. This is in marked contrast to the method of EP-0 079 139 where the concentration of the immobilised reagent is finite, and relatively low, so that the quantity of label bound to the duplex is equally low and is determined not by the concentration in the sample of the sequence to be detected, which is usually far in excess of the quantity of the immobilised reagent, but by the quantity of the immobilised agent. In the method of the present invention a far higher proportion of the sequence to be detected present in the sample is hybridised and consequently labelled. Not only that, but as already indicated, there is a substantial and surprising increase in the efficiency of the hybridisation reaction using beads as the carrier as opposed to a filter or membrane, e.g. of cellulose nitrate.

The sample used in the method of this invention can be a purified and/or fractionated sample containing the polynucleotide fragment or fragments to be identified in single-stranded form, but it is a major advantage of this invention that the sequence to be detected does not have to be separated from the sample prior to labelling as in the Southern blotting technique. It is therefore possible in accordance with the present invention to detect a given polynucleotide sequence in a crude mixture of polynucleotide fragments obtained by the digestion of the original sample, e.g. a clinical sample containing DNA or RNA, with an appropriate restriction endonuclease and subsequent denaturation to reduce the restriction fragments to singlestranded form.

Following hybridisation the Sephacryl® beads carrying the labelled hybridisation duplex are separated, e.g. by centrifuging and washing, from unbound excess label and the presence of bound label detected in the appropriate manner, e.g. by a scintillation counter in the case of a radioactive label, or by enzymatic activity in the case of an enzyme label, or light detecting means in the case of a fluorescent or chemiluminescent label.

The method of the present invention has a wide range of applications arising from the ability rapidly to identify particular nucleic acid (DNA or RNA) sequences in a sample. Thus it can be used to identify pathogenic micro-organisms in a sample, genetic abnormalities, etc. as well as simply a gene mapping exercise. A particular application given by way of example is in the identification of sickle cell disease.

3

Sickle cell disease is caused by a single base mutation (A → T) in the sixth codon of the human β-globin gene. This change from GAG to GTG in the sixth codon, besides altering the properties of the resultant haemoglobin coded for by the gene with valine being incorporated into the protein rather than glutamine, also results in the loss of the Dde I and Mst II recognition sites at the 5' end of the β-globin gene. This is diagramatically illustrated in the accompanying drawing which is a restriction map of Dde I and Hinf I sites at the 5' end of the β-globin gene. Dde I digestion of abnormal (sickle cell) β-globin gene will therefore produce a single 381 bp fragment comprising both Fragments B and C in a single continuous length, as opposed to two separate fragments, one of 201 bp (Fragment B) and 180 bp (Fragment C), that will be produced by Dde I digestion of the normal β-globin gene. Using the techniques already described to immobilise one of Fragment B or Fragment C onto polymer beads and to label the other, two reagents are provided capable of rapidly detecting the presence of abnormal sickle cell gene in a sample. Thus digestion of normal β-globin gene with Dde I will produce two fragments neither of which is capable of forming a "sandwich" with the two reagents. On the other hand, digestion of abnormal (sickle cell) β-globin gene with Dde I will produce a single fragment which is capable of forming a "sandwich" with the two reagents. When the Dde I digest is contacted with the two reagents under hybridisation conditions (i.e. after denaturation of the digest) the abnormal (sickle cell) β-globin gene will result in binding of the label, and hence a positive signal, whereas the normal β-globin gene will not.

In a practical experiment, the above procedure has been mimicked by digesting plasmid β-F5 with Hinf I. Plasmid β-F5 contains the 1.9 kb Bam HI restriction fragment of the human β-globin gene cloned into the single Bam HI site of the vector pAT 153 (Jackson et al., (1981), Eur. J. Biochem. 121, 27 - 31). Digestion of the plasmid β-F5 with Hinf I produced a 341 bp fragment (Fragment A) which was recovered from the product of digestion by electrophoresis and electroelution. This 341 bp fragment (Fragment A), as will be seen, comprises a substantial overlap (175 bp) with the 180 bp fragment (Fragment C), although not the whole of the 180 bp fragment because of the slightly different locations of the Dde I and Hinf I restriction sites. Nevertheless for the purpose of sandwich hybridisation with the 381 bp fragment the difference between the 175 bp sequence contained in Fragment A and the full 180 bp fragment (Fragment C) is immaterial, since the sequence is the same, and it is that which is important, not the actual length of the fragment.

After denaturation the 341 kb fragment was immobilised onto macroporous cellulosic polymer beads (Sephacryl® S500) using the diazotisation method described by Seed B. in (1982) Nucleic Acids Res. 10, 1799 - 1810.

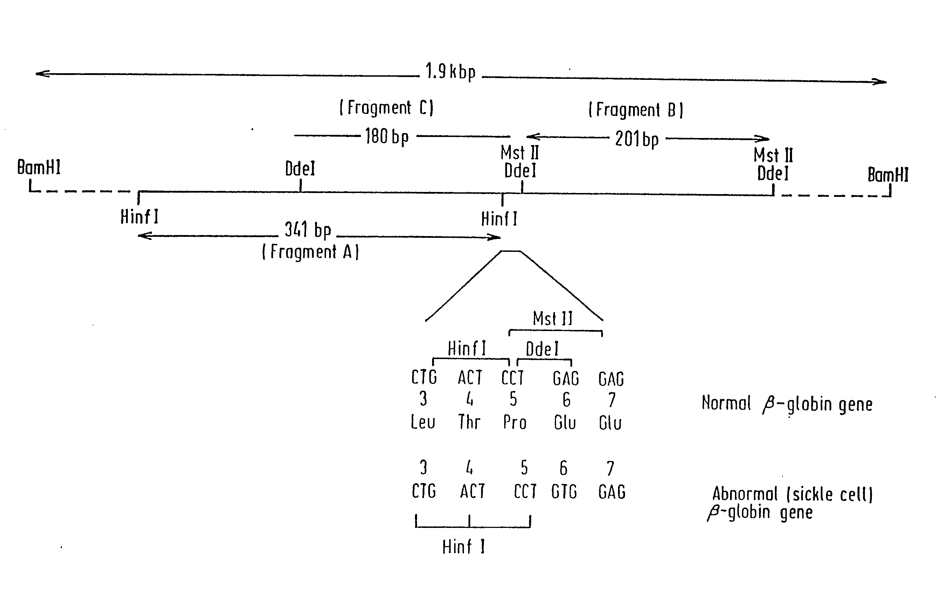

In a second operation plasmid β-F5 was digested with Mst II to produce a 201 bp fragment (Fragment B, see Figure 1) which was likewise recovered from the digest. Fragment B was then labelled with $^{32}P$ by nick translation using the technique of Rigby et al., (1977) J. Mol. Biol. 113, 237 - 251, to a specific activity of 1.5 x $10^7$ cpm μg$^{-1}$.

In two further operations plasmid β-F5 was digested with Dde I and Bam HI. Digestion with Dde I produces short fragments not capable of "sandwich" hybridisation with Fragments A and B, whereas digestion with Bam HI produces one long fragment which is capable of "sandwich" hybridisation. In each case digestion was followed by denaturation to provide single-stranded DNA fragments.

Prior to sandwich hybridisation by the technique of the present invention the immobilised Fragment A was prehybridised overnight at 65°C in 1 ml. 40 mM PIPES pH 6.5, 1 mM EDTA 0.6 M NaCl, 0.1 % SDS and 250 μg ml$^{-1}$ sonicated denatured salmon sperm DNA. Following prehybridisation, sandwich hybridisation was carried out by mixing either the denatured Dde I or Bam HI β-F5 digests with immobilised 341 bp Fragment A and labelled 201 bp Fragment B in 50 μl of the same buffer and allowing the mixture to stand overnight. The beads were then separated, washed three times for 10 minutes each at 65°C. with 1 ml of the same buffer but without the salmon sperm DNA and then counted. The results are shown in Table 1. These clearly show that with amounts of plasmid β-F5 of 100 attomoles or over, a clear distinction can be drawn between the Bam HI digest and the Dde I digest. In the figures given in Table 1 the low background count obtained when either the "sandwich filling" was omitted or replaced by sonicated calf thymus DNA has not been subtracted.

**Table 1**

| Amount of restricted β-F5 plasmid (attomoles) | cpm on beads when enzyme is Bam HI | cpm on beads when enzyme is Dde I |
|---|---|---|
| 10.000 | 552 | 63 |
| 5.000 | 748 | 70 |
| 2.000 | 482 | 65 |
| 1.000 | 266 | 71 |
| 500 | 196 | 44 |
| 100 | 107 | 62 |
| 80 | 90 | 61 |
| 60 | 82 | 60 |
| 40 | 62 | 57 |
| 20 | 75 | 60 |

In a further practical experiment, the above sandwich hybridisation using the immobilised 341 bp Fragment A and the labelled 201 bp Fragment B has been repeated using a Dde I digest of 60 attomoles of human DNA both from a normal patient and from a homozygote sickle patient. The results were unequivocal. After background substraction, a typical experiment gave 441 counts (10 minutes) for sickle DNA and 153 counts for normal DNA illustrating and confirming the effectiveness of the method of the invention in identifying the presence of sickle DNA.

By mixing the Bam HI and Dde I digests of β-F5 it is possible also to mimic the case of the sickle heterozygote. In such an experiment, counts (10 minutes) were obtained using 100 attomoles plasmid DNA as follows: normal 128; heterozygote 296; homozygote 745; thus indicating that the method can be quantitated to identify heterozygotes as well as homozygotes.

Im summary, the method of the present invention has the following advantages over present technology:

1. It is quicker. Electrophoresis, blotting and autoradiography are eliminated. The sandwich hybridisation can be carried out in as little as 8 hours; the separation of beads by gravity, centrifuge or magnetically takes seconds; and quantitation in a scintillation counter takes a few minutes. In practice results have been obtained within 48 hours of taking blood and the indications are that substantial reductions will be possible in the future.
2. It is extremely easy to automate and hence to process a large number of samples.
3. It is readily adaptable to other methods of DNA labelling.
4. It works as well with short restriction fragments as with longer ones and is readily adaptable for the use of oligonucleotides. This greatly enlarges the number of potential applications.
5. Whereas membranes have a (low) finite capacity for binding DNA, larger quantities of DNA can be readily handled in the present procedure by simply increasing the quantity of immobilised and labelled DNA's. This offers the possibility of either increasing the sensitivity of the assay or of using a labelled DNA of lower specific activity or of reducing the hybridisation time still further.

These advantages will now permit the rapid adoption of recombinant DNA technology to routine laboratories for the identification and characterisation of bacteria, viruses, and genetic disorders in humans, animals and plants.

## Claims

1. A method for the detection of a given nucleotide sequence in a target polynucleotide, which comprises contacting the target polynucleotide under hybridisation conditions with (a) an immobilised polynucleotide comprising a single-stranded nucleotide sequence complementary to, and hybridisable under said conditions, with a first section of said sequence to be detected, said polynucleotide being immobilised onto a particulate carrier material comprising beads of synthetic resin having said polynucleotide attached thereto, and (b) a labelled polynucleotide probe comprising a single-stranded nucleotide sequence complementary to, and hybridisable under said conditions, with a second and different section of the sequence to be detected, labelled with a marker, thereby to form an immobilised hybridisation duplex comprising the immobilised polynucleotide, the target polynucleotide and the labelled polynucleotide attached to said beads, separating the beads carrying the immobilised duplex from the reaction medium and determining the presence of the marker on the separated beads, *characterised in that* the immobilised polynucleotide, comprises a polynucleotide containing said single-stranded complementary nucleotide sequence covalently bonded to solid beads of N,N'-methylene bisacrylamide cross-linked allyl dextran (Sephacryl®), said beads having a size in the range 5 to 50 μm.

2. A method according to claim 1, wherein the immobilised polynucleotide is covalently bonded to the resin beads by a diazotisation reaction between reactive groups on the resin and free amino groups on the polynucleotide.

3. A method according to claim 1, wherein the immobilised polynucleotide is covalently bonded to the resin particles or beads by carbodiimide coupling between the phosphate groups at the 5' end of the polynucleotide and reactive groups on the resin.

4. A method according to claim 1, 2 or 3, wherein the polynucleotide probe comprises a radioactive marker or label.

5. A method according to claim 1, 2 or 3, wherein the polynucleotide probe comprises an enzyme marker or label.

6. A method according to any one of claims 1 - 5, wherein the immobilised polynucleotide and the polynucleotide probe are restriction endonuclease polynucleotide fragments.

7. A method according to claim 6, as applied to the detection of the abnormal human (sickle cell) β-globin gene, which comprises digesting the gene with a Dde I restriction endonuclease to produce a restriction digest containing as a component a 381 bp restriction fragment containing the abnormal sixth codon which is a characteristic of the abnormal gene, contacting the digest containing that fragment under hybridisation conditions with (a) said immobilised single-stranded polynucleotide covalently bonded to solid resin beads, said single-stranded polynucleotide being hybridisable with a first section of said fragment, and (b) with a labelled single-stranded polynucleotide probe hybridisable with a second section of said fragment, wherein one of said single-stranded polynucleotide probe and said labelled single-stranded polynucleotide is or comprises a 180 bp polynucleotide sequence obtainable by Dde I digestion of normal human β-globin gene and complementary to a first section of said 381 bp fragment, and the other is or comprises a 201 bp fragment obtainable by Dde I digestion of normal β-globin gene and complementary to a second, different, section of said 381 bp fragment, separating the beads carrying the resulting immobilised hybridisation duplex comprising the immobilised single-stranded polynucleotide, the said restriction fragment, and said labelled polynucleotide probe, and determining the presence of the marker on the separated duplex.

8. A method according to claim 7, wherein the said 180 bp polynucleotide sequence is replaced by a 341 bp fragment containing a sequence of 175 bp identical to that of the first 175 bp of said 180 bp fragment, said 341 bp fragment being obtainable by Hinf I digestion of normal human β-globin gene.

9. A method according to claim 8, wherein the said 341 bp fragment is obtained by the Hinf I digestion of plasmid β-F5, plasmid β-F5 itself being obtained by inserting the human β-globin gene into the Bam H1 site of vector pAT153.

10. A method according to claim wherein the said 201 bp fragment is also obtained from plasmid β-F5, but in this case by digestion of the plasmid with Mst II.

11. A diagnostic kit for the detection of a given nucleotide sequence in a target polynucleotide, comprising as a first component an immobilised polynucleotide reagent comprising solid beads of a synthetic resin having a polynucleotide comprising a single-stranded polynucleotide sequence complementary to a first section of the nucleotide sequence to be detected covalently bonded to said particles or beads, and as a second component, a polynucleotide probe comprising a single-stranded nucleotide sequence complementary to a second, different, section of the nucleotide sequence to be detected, and labelled with a marker, *characterised in that* said beads consist of N,N'-methylene bisacrylamide cross-linked allyl dextran (Sephacryl®), said beads having a size in the range 5 to 50 μm.

12. A diagnostic kit according to claim 11 for the detection of the abnormal human (sickle cell) β-globin gene, *characterised in that* one of said single stranded polynucleotide sequences is or comprises a 180 bp polynucleotide sequence obtainable by Dde I digestion of normal human β-globin gene, and the other is or comprises a 201 bp fragment obtainable by Dde I digestion of normal β-globin gene.

13. A kit according to claim 12, modified in that the said 180 bp polynucleotide sequence is replaced by a 341 bp fragment obtainable by Hinf I digestion of normal human β-globin gene, said 341 bp fragment containing a sequence of 175 bp identical to the first 175 bp of said 180 bp fragment.

14. A kit according to claim 13, wherein said 341 bp fragment is a product obtained by Hinf I digestion of plasmid β-F5.

15. A kit according to claim 14, wherein said 201 bp fragment is a product obtained by Mst II digestion of plasmid β-F5.

16. A kit according to any one of claims 10 - 15 wherein the said marker is an enzyme.

17. A kit according to claim 16, the kit also contains, as a third component, means for detecting the enzyme marker, said means comprising a substrate for said enzyme.

## Patentansprüche

1. Verfahren zum Erfassen einer vorgegebenen Nukleotidsequenz in einem Polynukleotidtarget mit Inkontaktbringen des Polynukleotidtargets unter Hybridisierungsbedingungen mit (a) einem immobilisierten Polynukleotid, welches eine einzelsträngige Nukleotidsequenz aufweist, welche komplementär zu einem ersten Abschnitt der Sequenz, die er faßt werden soll, ist und unter den genannten Bedingungen hybridisierbar ist, wobei das Polynukleotid auf einem besonderen Trägermaterial immobilisiert ist, welches Kunstharzkügelchen aufweist, an denen das Polynukleotid angebracht ist, und (b) einer markierten Polynukleotidsonde, die eine einzelsträngige Nukleotidsequenz aufweist, welche komplementär zu einem zweiten und verschiedenen Abschnitt der zu erfassenden Sequenz ist und unter den genannten Bedingungen hybridisierbar ist, markiert mit einem Markiermittel, um dadurch eine immobilisierte Hybridisierungsduplex zu bilden, welches das immobilisierte Polynukleotid aufweist, wobei das Polynukleotidtarget und das markierte Polynukleotid an den Kügelchen angebracht sind, Abscheiden der Kügelchen, welche die immobilisierte Duplex tragen, aus dem Reaktionsmedium und Feststellen des Vorhandenseins des Markiermittels auf den abgeschiedenen Kügelchen, *dadurch gekennzeichnet*, daß das immobilisierte Polynukleotid ein Polynukleotid aufweist, welches die einsträngige komplementäre Nukleotidsequenz kovalent gebunden an festen Kügelchen von N,N'-Methylenbisacrylamid vernetzt mit Allyldextran (Sephacryl®) enthält, wobei die Kügelchen eine Größe im Bereich von 5 bis 50 μm haben.

2. Verfahren nach Anspruch 1, wobei das immobilisierte Polynukleotid durch eine Diazotierungsreaktion zwischen reaktionsfähigen Gruppen des Kunstharzes und freien Aminogruppen auf dem Polynukleotid kovalent an die Kunstharzkügelchen gebunden ist.

3. Verfahren nach Anspruch 1, wobei das immobilisierte Polynukleotid durch Zyanamidkopplung zwischen den

6

Phosphatgruppen am 5'-Ende des Polynukleotids und reaktionsfähigen Gruppen auf dem Kunstharz kovalent an die Kunstharzpartikel gebunden ist.

4. Verfahren nach Anspruch 1, 2 oder 3, wobei die Polynukleotidsonde ein radioaktives Markiermittel aufweist.

5. Verfahren nach Anspruch 1, 2 oder 3, wobei die Polynukleotidsonde ein Enzym-Markiermittel aufweist.

6. Verfahren nach einem der Ansprüche 1 bis 5, wobei das immobilisierte Polynukleotid und die Polynukleotidsonde Restriktionsendonuklease-Polynukleotidbruchstücke sind.

7. Verfahren nach Anspruch 6, angewandt auf die Erfassung des anomalen menschlichen (Sichelzell) β-Globingenes, welches Ordnen bzw. Aufschließen des Genes mit einer Dde I Restriktionsendonuklease aufweist, um einen Restriktionsaufschluß herzustellen, der als eine Komponente ein 381 bp Restriktionsfragment enthält, das die anomale sechste Folge enthält, die ein Charakteristikum dem anomalen Genes ist, Inkontaktbringen des Aufschlusses, welcher dieses Fragment enthält, unter Hybridisierungsbedingungen mit (a) dem kovalent an feste Harzkügelchen gebundenen, immobilisierten einsträngigen Polynukleotid, wobei das einsträngige Polynukleotid mit einem ersten Abschnitt des Fragmentes hybridisierbar ist, und (b) mit einer markierten einsträngigen Polynukleotidsonde, die mit einem zweiten Abschnitt des Fragmentes hybridisierbar ist, wobei die einsträngige Polynukleotidsonde oder das markierte einsträngige Polynukleotid eine 180 bp Polynukleotidfolge ist oder eine solche aufweist, welche man durch Dde I Aufschluß eines normalen menschlichen β-Globingenes erhält und zu einem ersten Abschnitt des erwähnten 381 bp-Fragmentes komplementär ist, wobei das jeweils andere ein 201 bp-Fragment ist oder ein solches aufweist, welches durch Dde I Aufschluß des normalen β-Globingenes erhältlich und zu einem zweiten, anderen Abschnitt des erwähnten 381 bp-Fragmentes komplementär ist, Abscheiden der Kügelchen, welche die resultierende immobilisierte Hybridisierungsduplex tragen, welches das immobilisierte einzelsträngige Polynukleotid, das Restriktionsfragment und die markierte Polynukleotidsonde aufweist und Bestimmen des Vorhandenseins des Markierelementes auf der abgeschiedenen Duplex.

8. Verfahren nach Anspruch 7, wobei die 180 bp Polynukleotidfolge durch ein 341 bp-Fragment ersetzt wird, welches eine Folge von 175 bp enthält, die identisch mit den ersten 175 bp des 180 bp-Fragmentes ist, wobei das 341 bp-Fragment durch Hinf I Aufschluß normalen menschlichen β-Globingens erhältlich ist.

9. Verfahren nach Anspruch 8, wobei das 341 bp-Fragment durch Hinf I Aufschluß von Plasmid β-F5 erhalten wird, wobei das Plasmid β-F5 selbst durch Einsetzen des menschlichen β-Globingenes an den Bam H1 Platz des Vektors pAT 153 erhalten wurde.

10. Verfahren nach Anspruch 9, wobei das 201 bp-Fragment ebenfalls von dem Plasmid β-F5 erhalten wurde, jedoch in diesem Fall durch Aufschluß des Plasmides mit Mst II.

11. Diagnosesatz für die Erfassung einer vorgegebenen Nukleotidsequenz in einem Polynukleotidtarget, mit einem immobilisierten Polynukleotidreagens als erster Komponente, welches feste Kügelchen eines Kunstharzes aufweist, das ein Polynukleotid hat, welches eine einsträngige Polynukleotidfolge aufweist, die komplementär zu einem ersten Abschnitt der zu erfassenden Nukleotidfolge ist und die kovalent an die Teilchen oder Kügelchen gebunden ist, und als zweite Komponente eine Polynukleotidsonde aufweist, die eine einsträngige Nukleotidfolge hat, die zu einem zweiten anderen Abschnitt der zu erfassenden Nukleotidfolge komplementär ist und mit einem Markierungsmittel markiert ist, *dadurch gekennzeichnet*, daß die Kügelchen aus N,N'-Methylenbisacrylamid bestehen, welches mit Allyldextran vernetzt ist (Sephacryl®), wobei die Kügelchen eine Größe im Bereich von 5 bis 50 µm haben.

12. Diagnosesatz nach Anspruch 11 zur Erfassung des anomalen menschlichen (Sichelzellen) β-Globingenes, *dadurch gekennzeichnet*, daß eine der einsträngigen Polynukleotidfolgen eine 180 bp Nukleotidfolge ist oder eine solche aufweist, welche durch Dde I-Aufschluß normalen menschlichen β-Globingenes erhältlich ist und die andere ein 201 bp-Fragment ist oder ein solches aufweist, welches durch Dde I Aufschluß normalen β-Globingenes erhältlich ist.

13. Satz nach Anspruch 12, dadurch modifiziert, daß die 180 bp Polynukleotidsequenz durch ein 341 bp-Fragment ersetzt wird, welches durch Hinf I-Aufschluß normalen menschlichen β-Globingenes erhältlich ist, wobei das 341 bp-Fragment eine Folge von 175 bp enthält, die identisch mit den ersten 175 bp des 180 bp-Fragmentes ist.

14. Satz nach Anspruch 13, wobei das 341 bp-Fragment ein Produkt ist, welches durch Hinf I-Aufschluß des Plasmides β-F5 erhalten wurde.

15. Satz nach Anspruch 14, wobei das 201 bp Fragment ein Produkt ist, welches durch Mst II Aufschluß des Plasmides β-F5 erhalten wurde.

16. Satz nach einem der Ansprüche 10 bis 15, wobei das Markiermittel ein Enzym ist.

17. Satz nach Anspruch 16, wobei dieser Satz zusätzlich als dritte Komponente Mittel zum Erfassen des Enzymmarkiermittels enthält, wobei diese Mittel ein Substrat für das Enzym aufweisen.

**Revendications**

1. Procédé de détection d'une séquence déterminée de nucléotides dans un polynucléotide cible, lequel consiste à mettre en contact le polynucléotide cible dans des conditions d'hybridisation avec (a) un polynucléotide immobilisé comprenant une séquence mono-brin de nucléotides complémentaire et hybridisable dans lesdites conditions à une première section de ladite séquence à détecter, ledit polynucléotide étant immobilisé sur un matériau support particulaire comportant des billes de résine synthétique ayant ledit polynucléotique fixé à celles-ci, et (b) une sonde marquée de polynucléotide comprenant une séquence mono-brin de nucléotides complémentaire et hybridisable dans lesdites conditions à une seconde section différente de la séquence à détecter, marquée avec un marqueur, de façon à former un duplex d'hybridisation immobilisé comprenant le polynucléotide immobilisé, le polynucléotide cible et le polynucléotide marqué fixé auxdites billes, à séparer les billes portant le duplex immobilisé du milieu réactionnel et à déterminer la présence du marqueur sur les billes séparées, *caractérisé en ce que* le polynucléotide immobilisé comprend un polynucléotide contenant ladite séquence mono-brin de nucléotides complémentaire liée de manière covalente à des billes solides d'allyldextrane réticulé au N,N'-méthylène bisacrylamide (Séphacryl®), lesdites billes ayant une taille dans la gamme de 5 à 50 micromètres.

2. Procédé suivant la revendication 1, dans lequel le polynucléotide immobilisé est lié de manière covalente aux billes de résine par réaction de diazotation entre des groupes réactifs sur la résine et des groupes amino libres du polynucléotide.

3. Procédé suivant la revendication 1, dans lequel le polynucléotide immobilisé est lié de manière covalente aux billes ou particules de résine par couplage à la carbodiimide entre les groupes phosphate en position d'extrémité 5' du polynucléotide et des groupes réactifs sur la résine.

4. Procédé suivant la revendication 1, 2 ou 3, dans lequel la sonde polynucléotide comporte un marqueur ou indicateur radioactif.

5. Procédé suivant la revendication 1, 2 ou 3, dans lequel la sonde polynucléotide comporte un marqueur ou indicateur enzymatique.

6. Procédé suivant l'une quelconque des revendications 1 à 5, dans lequel le polynucléotide immobilisé et la sonde polynucléotide sont des fragments polynucléotide d'endonucléase de restriction.

7. Procédé suivant la revendication 6, appliqué à la détection du gène anormal β-globine humaine (drépanocyte) lequel consiste à mettre en digestion le gène avec une endonucléase de restriction Dde I pour produire un digesté de restriction contenant en tant que composant un fragment de restriction 381 bp contenant le sixième codon anormal, caractéristique du gène anormal, à mettre en contact le digesté contenant ce fragment dans des conditions d'hybridisation avec (a) ledit polynucléotide immobilisé mono-brin lié de manière covalente aux billes de résine solide, ledit polynucléotide mono-brin étant hybridisable avec une première section dudit fragment, et (b) avec une sonde polynucléotide mono-brin marquée hybridisable avec une seconde section dudit fragment, dans lequel l'un de ladite sonde polynucléotide mono-brin et dudit polynucléotide mono-brin marqué est constitué de ou comprend une séquence polynucléotide 180 bp obtenable par digestion Dde I du gène normal β-globine humaine et complémentaire à une première section dudit fragment 381 bp, et l'autre est constitué de ou comprend un fragment 201 bp obtenable par digestion Dde I du gène normal β-globine et complémentaire à une seconde et différente section dudit fragment 381 bp, à séparer les billes portant le duplex d'hybridisation immobilisé résultant comprenant le polynucléotide immobilisé mono-brin, ledit fragment de restriction, et ladite sonde polynucléotide marquée, et à déterminer la présence du marqueur sur le duplex séparé.

8. Procédé suivant la revendication 7, dans lequel la dite séquence polynucléotide 180 bp est remplacée par un fragment 341 bp contenant une séquence de 175 bp identique à celle des premières 175 bp dudit fragment 180 bp, ledit fragment 341 bp étant obtenable par digestion Hinf I du gène normal de la β-globine humaine.

9. Procédé suivant la revendication 8, dans lequel ledit fragment 341 bp est obtenu par la digestion Hinf I du plasmide β-F5, le plasmide β-F5 étant lui-même obtenu par insertion du gène β-globine humaine dans le site Bam H1 du vecteur pAT153.

10. Procédé suivant la revendication 9, dans lequel ledit fragment 201 bp est obtenu également du plasmide β-F5, mais en ce cas par digestion du plasmide avec Mst II.

11. Kit de diagnostic pour la détection d'une séquence déterminée de nucléotides dans un polynucléotide cible, comportant comme premier composant un réactif polynucléotide immobilisé comprenant des billes solides d'une résine synthétique ayant un polynucléotide comprenant une séquence polynucléotide mono-brin complémentaire à une première section de la séquence de nucléotides à détecter et liée de manière covalente auxdites particules ou billes, et comme second composant, une sonde polynucléotide comprenant une séquence de nucléotides mono-brin complémentaire à une seconde et différente section de la séquence de nucléotides à détecter, et marquée avec un marqueur,

*caractérise en ce que* lesdites billes consistant en de l'allyl dextrane réticulé au N,N'-méthylène bisacrylamide (Sépha-cryl®), lesdites billes ayant une taille dans la gamme de 5 à 50 micromètres.

12. Kit de diagnostic selon la revendication 11 pour la détection du gène anormal β-globine humaine (drépanocyte), *caractérisé en ce que* l'une desdites séquences polynuclaotide mono-brin est constitué de ou comprend une séquence polynucléotide 180 bp obtenable par digestion Dde I du gène normal β-globine humaine, et l'autre est constituée de ou comprend un fragment 201 bp obtenable par digestion Dde I du gène normal β-globine.

13. Kit selon la revendication 12, modifié en ce que ladite séquence polynucléotide 180 bp est remplacée par un fragment 341 bp obtenable par digestion Hinf I du gène normal β-globine humaine, ledit fragment 341 bp contenant une séquence de 175 bp dudit fragment 180 bp.

14. Kit selon la revendication 13, dans lequel ledit fragment 341 bp est un produit obtenu par digestion Hinf I du plasmide β-F5.

15. Kit selon la revendication 14, dans lequel ledit fragment 201 bp est un produit obtenu par digestion Mst II du plasmide β-F5.

16. Kit selon d'une quelconque des revendications 10 à 15, dans lequel ledit marqueur est une enzyme.

17. Kit selon la revendication 16, le kit contenant en outre, en tant que troisième composant, un moyen de détection du marqueur enzymatique, ledit moyen comprenant un substrat pour ladite enzyme.

EP 0 205 532 B1